# EUROPEAN PATENT APPLICATION

(11) **EP 4 108 189 A1**
(43) Date of publication of application: **28.12.2022**
(21) Application number: 22180670.6
(22) Date of filing: 23.06.2022
(51) Int. Cl.: A61B 17/32, A61B 18/12, A61B 18/14, A61B 17/00, A61B 18/00

(54) **ENERGY BASED SURGICAL INSTRUMENTS AND SYSTEMS**

(30) Priority: 24.06.2021 US 202163214699 P; 01.04.2022 US 202217711157
(71) Applicant: Covidien LP, Mansfield, MA 02048 (US)
(72) Inventor: FAGAN, James, R, Boulder, 80301 (US); DROCHNER, Thomas, E, Boulder, 80301 (US); COWLEY, Matthew, S, Boulder, 80301 (US); VAN TOL, David, J, Boulder, 80301 (US); LYONS, Michael, B, Boulder, 80301 (US)
(74) Representative: Maschio, Antonio

(57) **Abstract**

A surgical system includes a housing, an ultrasonic transducer supported by the housing, an end effector assembly distally-spaced from the housing, a clamp lever, and a sensor. The end effector assembly includes an ultrasonic blade operably coupled to the ultrasonic transducer for receiving ultrasonic energy produced by the ultrasonic transducer, and a jaw member pivotable relative to the blade from an open position towards a clamping position for clamping tissue between the jaw member and the blade and imparting a clamping force to the clamped tissue. The clamp lever is operably coupled to the housing and the jaw member such that actuation of the clamp lever relative to the housing pivots the jaw member towards the blade. The sensor is configured to sense whether the clamp lever is sufficiently actuated so as to impart the clamping force to the clamped tissue.

## Description

### CROSS-REFERENCE TO RELATED APPLICATION

This application claims the benefit of, and priority to, U.S. Provisional Patent Application No. 63/214,699, filed on June 24, 2021, the entire contents of which are hereby incorporated herein by reference.

### FIELD

The present disclosure relates to energy based surgical instruments and, more particularly, to surgical instruments and systems incorporating ultrasonic and/or electrosurgical functionality to facilitate energy based tissue treatment.

### BACKGROUND

Ultrasonic surgical instruments and systems utilize ultrasonic energy, i.e., ultrasonic vibrations, to treat tissue. More specifically, ultrasonic surgical instruments and systems utilize mechanical vibration energy transmitted at ultrasonic frequencies to treat tissue. An ultrasonic surgical device may include, for example, an ultrasonic blade and a clamp mechanism to enable clamping of tissue against the blade. Ultrasonic energy transmitted to the blade causes the blade to vibrate at very high frequencies, which allows for heating tissue to treat tissue clamped against or otherwise in contact with the blade.

Electrosurgical instruments and systems conduct Radio Frequency (RF) energy through tissue to treat tissue. An electrosurgical instrument or system may be configured to conduct bipolar RF energy between oppositely charged electrodes and through tissue, e.g., tissue clamped between the electrodes or otherwise in contact therewith, to treat tissue. Alternatively or additionally, an electrosurgical instrument or system may be configured to deliver monopolar RF energy from an active electrode to tissue in contact with the electrode, with the energy returning via a remote return electrode device to complete the circuit.

### SUMMARY

As used herein, the term "distal" refers to the portion that is described which is further from an operator (whether a human surgeon or a surgical robot), while the term "proximal" refers to the portion that is being described which is closer to the operator. Terms including "generally," "about," "substantially," and the like, as utilized herein, are meant to encompass variations, e.g., manufacturing tolerances, material tolerances, use and environmental tolerances, measurement variations, and/or other variations, up to and including plus or minus 10 percent. Further, any or all of the aspects described herein, to the extent consistent, may be used in conjunction with any or all of the other aspects described herein.

Provided in accordance with aspects of the present disclosure is a surgical system including a housing, an ultrasonic transducer supported by the housing, an end effector assembly distally-spaced from the housing, a clamp lever, and a sensor. The end effector assembly includes an ultrasonic blade operably coupled to the ultrasonic transducer for receiving ultrasonic energy produced by the ultrasonic transducer, and a jaw member pivotable relative to the blade from an open position towards a clamping position for clamping tissue between the jaw member and the blade and imparting a clamping force to the clamped tissue. The clamp lever is operably coupled to the housing and the jaw member such that actuation of the clamp lever relative to the housing pivots the jaw member towards the blade. The sensor is configured to sense whether the clamp lever is sufficiently actuated so as to impart the clamping force to the clamped tissue.

In an aspect of the present disclosure, the surgical system further includes a generator operably coupled to the ultrasonic transducer and configured to control activation of the ultrasonic transducer to generate ultrasonic energy for transmission to the blade.

In another aspect of the present disclosure, the generator is communicatively coupled to the sensor and configured to control activation of the ultrasonic transducer based at least in part on feedback from the sensor indicating whether the clamp lever is sufficiently actuated.

In still another aspect of the present disclosure, at least one activation button configured for activation in each of a first state and a second state is provided. In such aspects, the generator may be configured to control activation of the ultrasonic transducer based at least in part on feedback from the sensor indicating whether the clamp lever is sufficiently actuated and the activation state of the at least one activation button.

In yet another aspect of the present disclosure, the generator is communicatively coupled to the sensor and configured to provide an output based on feedback from the sensor indicating whether the clamp lever is sufficiently actuated.

In still yet another aspect of the present disclosure, at least one of the jaw member or the blade is adapted to connect to a source of electrosurgical energy for supplying electrosurgical energy to tissue. The jaw member and the blade may be adapted to connect to the source of electrosurgical energy for conducting bipolar electrosurgical energy therebetween and through tissue and/or at least one of the jaw member or the blade may be adapted to connect to the source of electrosurgical energy for supplying monopolar electrosurgical energy through tissue to a remote return device.

Another surgical system provided in accordance with aspect of the present disclosure includes a housing, an ultrasonic transducer supported by the housing, an end effector assembly distally-spaced from the housing, a clamp jaw, a sensor, and a generator. The end effector assembly includes an ultrasonic blade operably coupled to the ultrasonic transducer for receiving ultrasonic energy produced by the ultrasonic transducer, and a jaw member pivotable relative to the blade from an open position towards a clamping position for clamping tissue between the jaw member and the blade and imparting a clamping force to the clamped tissue. The clamp lever is operably coupled to the housing and the jaw member such that actuation of the clamp lever relative to the housing pivots the jaw member towards the blade. The sensor is configured to sense whether the clamp lever is sufficiently actuated so as to impart the clamping force to the clamped tissue. The surgical generator is operably coupled to the ultrasonic transducer to control activation of the ultrasonic transducer to generate ultrasonic energy for transmission to the blade and communicatively coupled to the sensor. The generator is configured to at least one of provide an output based on feedback from the sensor indicating whether the clamp lever is sufficiently actuated, or control activation of the ultrasonic transducer based at least in part on feedback from the sensor indicating whether the clamp lever is sufficiently actuated.

In an aspect of the present disclosure, at least one activation button is provided. The at least one activation button is configured for activation in each of a first state and a second state. In such aspects, the generator may be configured to control activation of the ultrasonic transducer based at least in part on feedback from the sensor indicating whether the clamp lever is sufficiently actuated and the activation state of the at least one activation button.

In another aspect of the present disclosure, the generator is configured to supply electrosurgical energy to at least one of the jaw member or the blade for supplying the electrosurgical energy to tissue. In such aspects, the jaw member and the blade may be configured to conduct bipolar electrosurgical energy therebetween and through tissue and/or at least one of the jaw member or the blade may be configured to supply monopolar electrosurgical energy through tissue to a remote return device for return to the generator.

In yet another aspect of the present disclosure, the generator is configured to control activation of the ultrasonic transducer and the supply of electrosurgical energy based at least in part on feedback from the sensor indicating whether the clamp lever is sufficiently actuated.

### BRIEF DESCRIPTION OF THE DRAWINGS

The above and other aspects and features of the present disclosure will become more apparent in light of the following detailed description when taken in conjunction with the accompanying drawings wherein like reference numerals identify similar or identical elements.
FIG. 1 is a side view of a surgical system provided in accordance with the present disclosure including a surgical instrument, a surgical generator, and a return electrode device;
FIG. 2 is perspective view of another surgical system provided in accordance with the present disclosure including a surgical instrument incorporating an ultrasonic generator, electrosurgical generator, and power source therein;
FIG. 3 is a schematic illustration of a robotic surgical system provided in accordance with the present disclosure;
FIG. 4 is a longitudinal, cross-sectional view of a distal end portion of the surgical instrument of FIG. 1;
FIG. 5 is a transverse, cross-sectional view of the end effector assembly of the surgical instrument of FIG. 1; and
FIG. 6 is a transverse, cross-sectional view of another configuration of the end effector assembly of the surgical instrument of FIG. 1.

### DETAILED DESCRIPTION

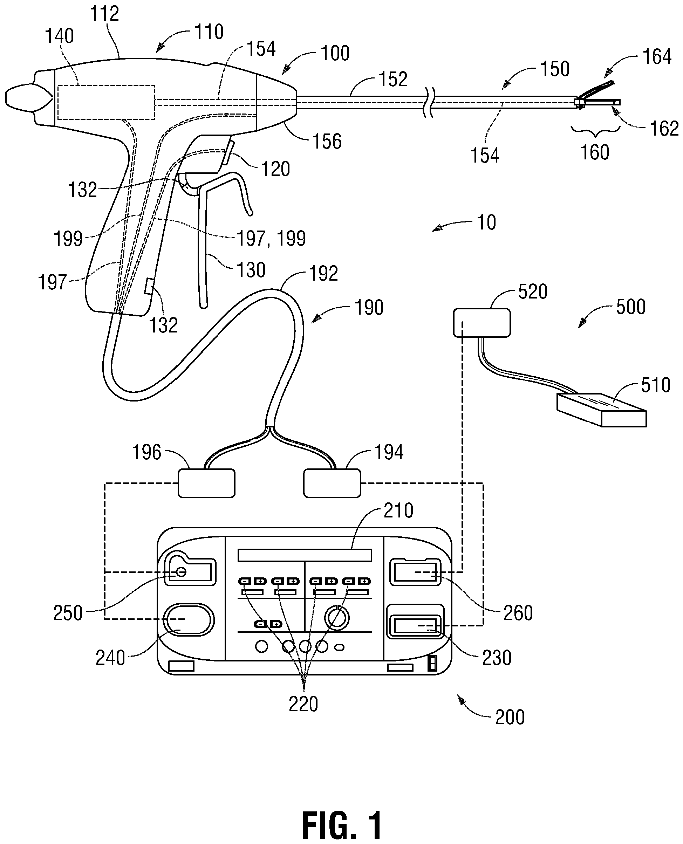

Referring to FIG. 1, a surgical system provided in accordance with aspects of the present disclosure is shown generally identified by reference numeral 10 including a surgical instrument 100, a surgical generator 200, and, in some aspects, a return electrode device 500, e.g., including a return pad 510. Surgical instrument 100 includes a handle assembly 110, an elongated assembly 150 extending distally from handle assembly 110, an end effector assembly 160 disposed at a distal end of elongated assembly 150, and a cable assembly 190 operably coupled with handle assembly 110 and extending therefrom for connection to surgical generator 200.

Surgical generator 200 includes a display 210, a plurality user interface features 220, e.g., buttons, touch screens, switches, etc., an ultrasonic plug port 230, a bipolar electrosurgical plug port 240, and active and return monopolar electrosurgical plug ports 250, 260, respectively. As an alternative to plural dedicated ports 230-260, one or more common ports (not shown) may be configured to act as any two or more of ports 230-260.

Surgical instrument 100 is configured to supply electrosurgical, e.g., Radio Frequency (RF), energy to tissue to treat tissue, e.g., in a monopolar configuration and/or a bipolar configuration, and/or to supply ultrasonic energy to tissue to treat tissue. Surgical generator 200 is configured to produce ultrasonic drive signals for output through ultrasonic plug port 230 to surgical instrument 100 (in aspects where surgical instrument 100 is configured to deliver ultrasonic energy) to activate surgical instrument 100 to supply ultrasonic energy and to provide electrosurgical energy, e.g., RF bipolar energy for output through bipolar electrosurgical plug port 240 and/or RF monopolar energy for output through active monopolar electrosurgical port 250 to surgical instrument 100 (in aspects where surgical instrument 100 is configured to deliver electrosurgical energy) to activate surgical instrument 100 to supply electrosurgical energy. Plug 520 of return electrode device 500 is configured to connect to return monopolar electrosurgical plug port 260 to return monopolar electrosurgical energy from surgical instrument 100 during monopolar electrosurgical use.

Continuing with reference to FIG. 1, handle assembly 110 includes a housing 112, an activation button 120, and a clamp lever 130. Housing 112 is configured to support an ultrasonic transducer 140. Ultrasonic transducer 140 may be permanently engaged within housing 112 or removable therefrom. Ultrasonic transducer 140 includes a piezoelectric stack or other suitable ultrasonic transducer components electrically coupled to surgical generator 200, e.g., via one or more of first electrical lead wires 197, to enable communication of ultrasonic drive signals to ultrasonic transducer 140 to drive ultrasonic transducer 140 to produce ultrasonic vibration energy that is transmitted along a waveguide 154 of elongated assembly 150 to blade 162 of end effector assembly 160 of elongated assembly 150, as detailed below. Feedback and/or control signals may likewise be communicated between ultrasonic transducer 140 and surgical generator 200. Ultrasonic transducer 140, more specifically, may include a stack of piezoelectric elements secured, under pre-compression between proximal and distal end masses or a proximal end mass and an ultrasonic horn with first and second electrodes electrically coupled between piezoelectric elements of the stack of piezoelectric elements to enable energization thereof to produce ultrasonic energy. However, other suitable ultrasonic transducer configurations, including plural transducers and/or non-longitudinal, e.g., torsional, transducers are also contemplated.

An activation button 120 is disposed on housing 112 and coupled to or between ultrasonic transducer 140 and/or surgical generator 200, e.g., via one or more of first electrical lead wires 197, to enable activation of ultrasonic transducer 140 in response to depression of activation button 120. In some configurations, activation button 120 may include an ON/OFF switch. In other configurations, activation button 120 may include multiple actuation switches to enable activation from an OFF state to different states corresponding to different activation settings, e.g., a first state corresponding to a first activation setting (such as a LOW power and/or tissue sealing setting) and a second state corresponding to a second activation setting (such as a HIGH power and/or tissue transection setting). In still other configurations, separate activation buttons may be provided, e.g., a first actuation button for activating a first activation setting and a second activation button for activating a second activation setting. Additional activation buttons, sliders, wheels, etc. are also contemplated to enable control of various different activation settings from housing 112.

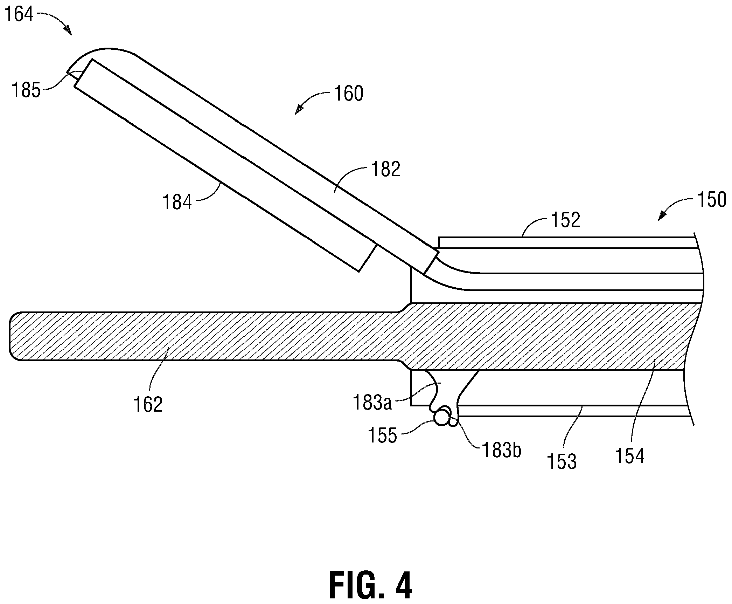

Elongated assembly 150 of surgical instrument 100 includes an outer drive sleeve 152, an inner support sleeve 153 (FIG. 4) disposed within outer drive sleeve 152, a waveguide 154 extending through inner support sleeve 153 (FIG. 4), a drive assembly (not shown), a rotation knob 156, and an end effector assembly 160 including a blade 162 and a jaw member 164. Rotation knob 156 is rotatable in either direction to rotate elongated assembly 150 in either direction relative to handle assembly 110. The drive assembly operably couples a proximal portion of outer drive sleeve 152 to clamp lever 130 of handle assembly 110. A distal portion of outer drive sleeve 152 is operably coupled to jaw member 164 and a distal end of inner support sleeve 153 (FIG. 4) pivotably supports jaw member 164. As such, clamp lever 130 is selectively actuatable, e.g., between an un-actuated position and a fully actuated position, to thereby move outer drive sleeve 152 about inner support sleeve 153 (FIG. 4) to pivot jaw member 164 relative to blade 162 of end effector assembly 160 from an open position towards a closed position for clamping tissue between jaw member 164 and blade 162. The configuration of outer and inner sleeves 152, 153 (FIG. 4) may be reversed, e.g., wherein outer sleeve 152 is the support sleeve and inner sleeve 153 (FIG. 4) is the drive sleeve. Other suitable drive structures as opposed to a sleeve are also contemplated such as, for example, drive rods, drive cables, drive screws, etc.

Referring still to FIG. 1, the drive assembly may be tuned to provide a jaw clamping force, or jaw clamping force within a jaw clamping force range, to tissue clamped between jaw member 164 and blade 162, such as described in U.S. Patent Application No. 17/071,263, filed on October 15, 2020, the entire contents of which are hereby incorporated herein by reference. Alternatively, the drive assembly may include a force limiting feature, e.g., a spring, whereby the clamping force applied to tissue clamped between jaw member 164 and blade 162 is limited to a particular jaw clamping force or a jaw clamping force within a jaw clamping force range, such as described in U.S. Patent No. 10,368,898, issued on August 6, 2019, the entire contents of which are hereby incorporated herein by reference.

Regardless of the particular configuration for jaw clamping force control, and even with the lack thereof, upon sufficient actuation of clamp lever 130, jaw member 164 is pivoted towards blade 162 to contact and clamp tissue between jaw member 164 and blade 162, whereby the tissue resists compression thereby providing an opposing force to the clamping force applied by jaw member 164. This point of sufficient actuation of clamp lever 130, wherein a clamp force is applied and a resistive force is provided, establishes or increases tension in the drive train (e.g., from clamp lever 130, through the drive assembly, to jaw member 164). Prior to reaching this point, clamp lever 130 provides no force or a negligible force to tissue and the drive train is less tensioned or substantially un-tensioned. The particular point of sufficient actuation may vary depending upon the size, position, and/or type of tissue clamped. Further, flexibilities, tolerances, and/or deflections in the drive train, e.g., in clamp lever 130, the drive assembly, and/or end effector assembly 160, may result in a disjunction between the position of clamp lever 130 and the position of jaw member 164 once the point of sufficient actuation is achieved. For example, where relatively large diameter tissue, e.g., greater than 7mm, is clamped between jaw member 164 and blade 162, clamp lever 130 may be actuated a relatively small amount to pivot jaw member 164 towards blade 162 before reaching the point of sufficient actuation (e.g., before the drive train is tensioned or further tensioned and before the clamping and resistive forces are provided). Thereafter, clamp lever 130 may move to a fully actuated position while jaw member 164 substantially remains in position, with flexibilities, tolerances, and/or deflections allowing for this disjunction such that minimal or no additional clamping force is imparted to the clamped tissue. On the other hand, where relatively small diameter tissue, e.g., less than or equal to 7mm, is clamped between jaw member 164 and blade 162, clamp lever 130 may be actuated a relatively larger amount or fully to pivot jaw member 164 towards blade 162 before the drive train is tensioned or further tensioned and the clamping and resistive forces are provided, with little to no further actuation of clamp lever 130 or disjunction between clamp lever 130 and jaw member 164 provided.

One or more sensors 132 are provided to sense that clamp lever 130 has been actuated at least to the point of sufficient actuation and, thus, to sense whether clamping force is applied to tissue clamped between jaw member 164 and blade 162. A sensor 132 may be disposed on clamp lever 130, a sensor 132 may be disposed on the fixed handle portion of housing 112, and/or a sensor 132 may be disposed in any other suitable position. The one or more sensors 132 may be similar or different from one another, may operate collectively or independently of one another, and may be any suitable sensors such as, for example, force sensors, strain gauges, contact sensors, proximity sensors, etc. configured to sense or enable determination based on sensed data, whether clamp lever 130 has been actuated at least to the point of sufficient actuation. With respect to position or proximity sensing, if clamp lever 130 is detected in the fully actuated position, it may be assumed that clamp lever 130 has been actuated at least to the point of sufficient actuation. With respect to force sensing or strain gauge sensing, if a force or strain on clamp lever 130 is detected above a threshold, it may be determined that the drive train is tensioned or further tensioned and, thus, that clamp lever 130 has been actuated at least to the point of sufficient actuation.

Sensor(s) 132 may communicate, e.g., via wired or wireless communication, with generator 200 to provide feedback thereto as to whether clamp lever 130 has been actuated at least to the point of sufficient actuation. As detailed below, generator 200, based upon the feedback received from the one or more sensor 132, in turn, controls the application of energy (e.g., enables/disables the application of energy, selects the modality(s) of energy to be applied, and/or the selects the parameters of energy delivery (e.g., HIGH vs LOW power)) based upon whether clamp lever 130 has been actuated at least to the point of sufficient actuation and/or other information such as, for example, the manner of actuation by the user. Alternatively or additionally, as also detailed below, generator 200 may provide an output to the user such as, for example, an audible output (e.g., a tone), a visual output (e.g., illumination of an LED of surgical instrument 100 or visual indicator on display 210), a haptic output (e.g., vibration of housing 112 of surgical instrument 100), and/or other suitable output based upon whether clamp lever 130 has been actuated at least to the point of sufficient actuation and/or other information such as, for example the manner of actuation by the user.

Continuing with reference to FIG. 1, waveguide 154, as noted above, extends from handle assembly 110 through inner sleeve 153 (FIG. 4). Waveguide 154 includes blade 162 disposed at a distal end thereof. Blade 162 may be integrally formed with waveguide 154, separately formed and subsequently attached (permanently or removably) to waveguide 154, or otherwise operably coupled with waveguide 154. Waveguide 154 and/or blade 162 may be formed from titanium, a titanium alloy, or other suitable electrically conductive material(s), although non-conductive materials are also contemplated. Waveguide 154 includes a proximal connector (not shown), e.g., a threaded male connector, configured for engagement, e.g., threaded engagement within a threaded female receiver, of ultrasonic transducer 140 such that ultrasonic motion produced by ultrasonic transducer 140 is transmitted along waveguide 154 to blade 162 for treating tissue clamped between blade 162 and jaw member 164 or positioned adjacent to blade 162.

Cable assembly 190 of surgical instrument 100 includes a cable 192, an ultrasonic plug 194, and an electrosurgical plug 196. Ultrasonic plug 194 is configured for connection with ultrasonic plug port 230 of surgical generator 200 while electrosurgical plug 196 is configured for connection with bipolar electrosurgical plug port 240 of surgical generator 200 and/or active monopolar electrosurgical plug port 250 of surgical generator 200. In configurations where generator 200 includes a common port, cable assembly 190 may include a common plug (not shown) configured to act as both the ultrasonic plug 194 and the electrosurgical plug 196.

Plural first electrical lead wires 197 electrically coupled to ultrasonic plug 194 extend through cable 192 and into handle assembly 110 for electrical connection to ultrasonic transducer 140 and/or activation button 120 to enable the selective supply of ultrasonic drive signals from surgical generator 200 to ultrasonic transducer 140 upon activation of ultrasonic energy. In addition, plural second electrical lead wires 199 are electrically coupled to electrosurgical plug 196 and extend through cable 192 into handle assembly 110. In bipolar configurations, separate second electrical lead wires 199 are electrically coupled to waveguide 154 and jaw member 164 (and/or different portions of jaw member 164) such that bipolar electrosurgical energy may be conducted between blade 162 and jaw member 164 (and/or between different portions of jaw member 164). In monopolar configurations, a second electrical lead wire 199 is electrically coupled to waveguide 154 such that monopolar electrosurgical energy may be supplied to tissue from blade 162. Alternatively or additionally, a second electrical lead wire 199 may electrically couple to jaw member 164 in the monopolar configuration to enable monopolar electrosurgical energy to be supplied to tissue from jaw member 164. In configurations where both bipolar and monopolar functionality are enabled, one or more of the second electrical lead wires 199 may be used for both the delivery of bipolar energy and monopolar energy; alternatively, bipolar and monopolar energy delivery may be provided by separate second electrical lead wires 199. One or more other second electrical lead wires 199 is electrically coupled to activation button 120 to enable the selective supply of electrosurgical energy from surgical generator 200 to waveguide 154 and/or jaw member 164 upon activation of electrosurgical energy.

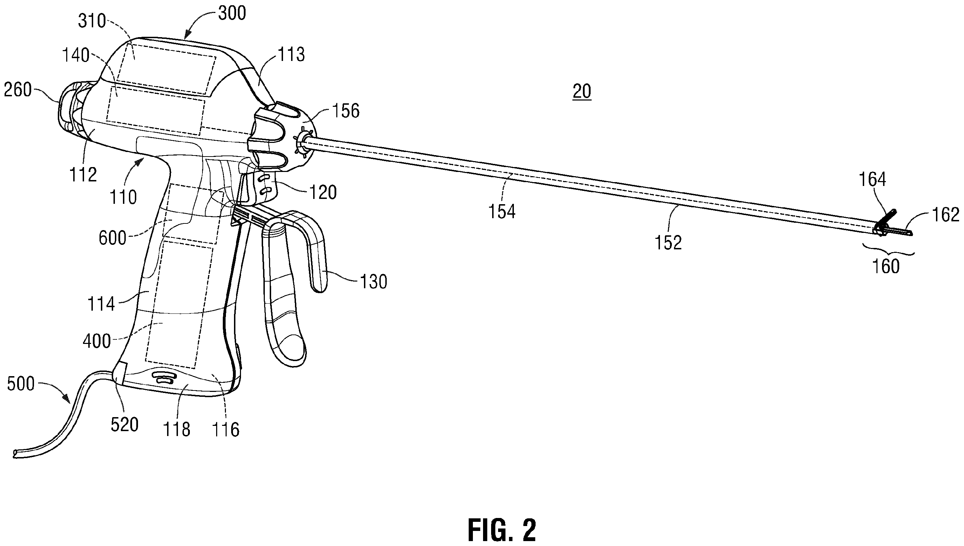

As an alternative to a remote generator 200, surgical system 10 may be at least partially cordless in that it incorporates an ultrasonic generator, an electrosurgical generator, and/or a power source, e.g., a battery, thereon or therein. In this manner, the connections from surgical instrument 100 to external devices, e.g., generator(s) and/or power source(s), is reduced or eliminated. More specifically, with reference to FIG. 2, another surgical system in accordance with the present disclosure is shown illustrated as a surgical instrument 20 supporting an ultrasonic generator 310, a power source (e.g., battery assembly 400), and an electrosurgical generator 600 thereon or therein. Surgical instrument 20 is similar to surgical instrument 100 (FIG. 1) and may include any of the features thereof except as explicitly contradicted below. Accordingly, only differences between surgical instrument 20 and surgical instrument 100 (FIG. 1) are described in detail below while similarities are omitted or summarily described.

Housing 112 of surgical instrument 20 includes a body portion 113 and a fixed handle portion 114 depending from body portion 113. Body portion 113 of housing 112 is configured to support an ultrasonic transducer and generator assembly ("TAG") 300 including ultrasonic generator 310 and ultrasonic transducer 140. TAG 300 may be permanently engaged with body portion 113 of housing 112 or removable therefrom.

Fixed handle portion 114 of housing 112 defines a compartment 116 configured to receive battery assembly 400 and electrosurgical generator 600 and a door 118 configured to enclose compartment 116. An electrical connection assembly (not shown) is disposed within housing 112 and serves to electrically couple activation button 120, ultrasonic generator 310 of TAG 300, and battery assembly 400 with one another when TAG 300 is supported on or in body portion 113 of housing 112 and battery assembly 400 is disposed within compartment 116 of fixed handle portion 114 of housing 112, thus enabling activation of surgical instrument 20 in an ultrasonic mode in response to appropriate actuation of activation button 120. Further, the electrical connection assembly or a different electrical connection assembly disposed within housing 112 serves to electrically couple activation button 120, electrosurgical generator 600, battery assembly 400, and end effector assembly 160 (e.g., blade 162 and jaw member 164 and/or different portions of jaw member 164) with one another when electrosurgical generator 600 and battery assembly 400 are disposed within compartment 116 of fixed handle portion 114 of housing 112, thus enabling activation of surgical instrument 20 to supply electrosurgical energy, e.g., bipolar RF energy, in response to appropriate actuation of activation button 120. To enable the supply of monopolar electrosurgical energy, plug 520 of return electrode device 500 may be configured to connect to surgical instrument 20 (electrosurgical generator 600 thereof, more specifically), to complete a monopolar circuit through tissue and between surgical instrument 20 (e.g., blade 162 and/or jaw member 164) and return electrode device 500.

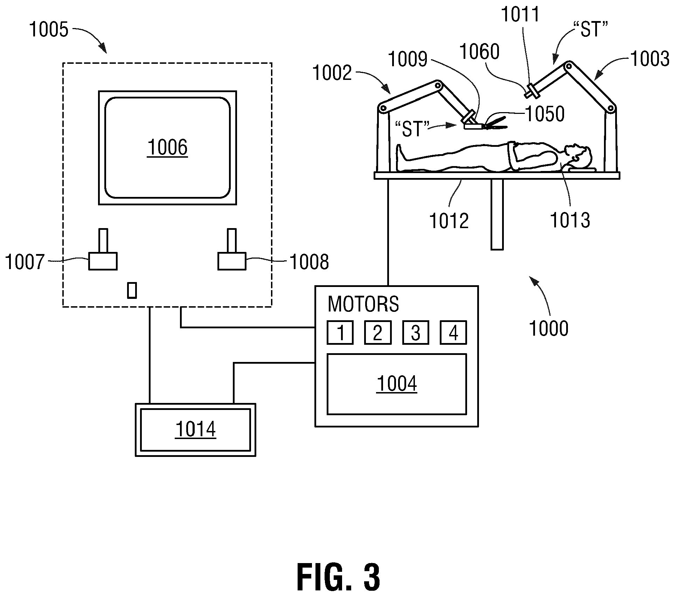

Turning to FIG. 3, a robotic surgical system in accordance with the aspects and features of the present disclosure is shown generally identified by reference numeral 1000. For the purposes herein, robotic surgical system 1000 is generally described. Aspects and features of robotic surgical system 1000 not germane to the understanding of the present disclosure are omitted to avoid obscuring the aspects and features of the present disclosure in unnecessary detail.

Robotic surgical system 1000 generally includes a plurality of robot arms 1002, 1003; a control device 1004; and an operating console 1005 coupled with control device 1004. Operating console 1005 may include a display device 1006, which may be set up in particular to display three dimensional images; and manual input devices 1007, 1008, by means of which a person (not shown), for example a surgeon, may be able to telemanipulate robot arms 1002, 1003 in a first operating mode. Robotic surgical system 1000 may be configured for use on a patient 1013 lying on a patient table 1012 to be treated in a minimally invasive manner. Robotic surgical system 1000 may further include a database 1014, in particular coupled to control device 1004, in which are stored, for example, pre-operative data from patient 1013 and/or anatomical atlases.

Each of the robot arms 1002, 1003 may include a plurality of members, which are connected through joints, and an attaching device 1009, 1011, to which may be attached, for example, a surgical tool "ST" supporting an end effector 1050, 1060. One of the surgical tools "ST" may be surgical instrument 100 (FIG. 1), surgical instrument 20 (FIG. 2), or any other suitable surgical instrument 20 configured for use in both an ultrasonic mode and one or more electrosurgical (bipolar and/or monopolar) modes, wherein manual actuation features, e.g., actuation button 120 (FIG. 1), clamp lever 130 (FIG. 1), etc., are replaced with robotic inputs. Further, sensor(s) for sensing the robotic inputs similarly as detailed above with respect to sensing clamp lever 130 (FIG. 1) may be provided. Robotic surgical system 1000 may include or be configured to connect to an ultrasonic generator, an electrosurgical generator, and/or a power source. The other surgical tool "ST" may include any other suitable surgical instrument, e.g., an endoscopic camera, other surgical tool, etc. Robot arms 1002, 1003 may be driven by electric drives, e.g., motors, that are connected to control device 1004. Control device 1004 (e.g., a computer) may be configured to activate the motors, in particular by means of a computer program, in such a way that robot arms 1002, 1003, their attaching devices 1009, 1011, and, thus, the surgical tools "ST" execute a desired movement and/or function according to a corresponding input from manual input devices 1007, 1008, respectively. Control device 1004 may also be configured in such a way that it regulates the movement of robot arms 1002, 1003 and/or of the motors.

Referring to FIGS. 4-6, end effector assembly 160 of surgical instrument 100 of surgical system 10 (FIG. 1) is detailed, although the aspects and features of end effector assembly 160 may similarly apply, to the extent consistent, to surgical instrument 20 (FIG. 2) and/or any other suitable surgical instrument or system. End effector assembly 160, as noted above, includes blade 162 and jaw member 164. Blade 162 may define a linear configuration, may define a curved configuration, or may define any other suitable configuration, e.g., straight and/or curved surfaces, portions, and/or sections; one or more convex and/or concave surfaces, portions, and/or sections; etc. With respect to curved configurations, blade 162, more specifically, may be curved in any direction relative to jaw member 164, for example, such that the distal tip of blade 162 is curved towards jaw member 164, away from jaw member 164, or laterally (in either direction) relative to jaw member 164. Further, blade 162 may be formed to include multiple curves in similar directions, multiple curves in different directions within a single plane, and/or multiple curves in different directions in different planes. In addition, blade 162 may additionally or alternatively be formed to include any suitable features, e.g., a tapered configuration, various different cross-sectional configurations along its length, cut outs, indents, edges, protrusions, straight surfaces, curved surfaces, angled surfaces, wide edges, narrow edges, and/or other features.

Blade 162 may define a polygonal, rounded polygonal, or any other suitable cross-sectional configuration(s). Waveguide 154 or at least the portion of waveguide 154 proximally adjacent blade 162, may define a cylindrical shaped configuration. Plural tapered surfaces (not shown) may interconnect the cylindrically shaped waveguide 154 with the polygonal (rounded edge polygonal, or other suitable shape) configuration of blade 162 to define smooth transitions between the body of waveguide 154 and blade 162.

Blade 162 may be wholly or selectively coated with a suitable material, e.g., a nonstick material, an electrically insulative material, an electrically conductive material, combinations thereof, etc. Suitable coatings and/or methods of applying coatings include but are not limited to Teflon^{®}, polyphenylene oxide (PPO), deposited liquid ceramic insulative coatings; thermally sprayed coatings, e.g., thermally sprayed ceramic; Plasma Electrolytic Oxidation (PEO) coatings; anodization coatings; sputtered coatings, e.g., silica; ElectroBond^{®} coating available from Surface Solutions Group of Chicago, IL, USA; or other suitable coatings and/or methods of applying coatings.

Continuing with reference to FIGS. 4-6, blade 162, as noted above, in addition to receiving ultrasonic energy transmitted along waveguide 154 from ultrasonic transducer 140 (FIG. 1), is adapted to connect to generator 200 (FIG. 1) to enable the supply of RF energy to blade 162 for conduction to tissue in contact therewith. In bipolar configurations, RF energy is conducted between blade 162 and jaw member 164 (or between portions of jaw member 164 and/or blade 162) and through tissue disposed therebetween to treat tissue. In monopolar configurations, RF energy is conducted from blade 162, serving as the active electrode, to tissue in contact therewith and is ultimately returned to generator 200 (FIG. 1) via return electrode device 500 (FIG. 1), serving as the passive or return electrode.

Jaw member 164 of end effector assembly 160 includes more rigid structural body 182 and more compliant jaw liner 184. Structural body 182 may be formed from an electrically conductive material, e.g., stainless steel, and/or may include electrically conductive portions. Structural body 182 includes a pair of proximal flanges 183a that are pivotably coupled to the inner support sleeve 153 via receipt of pivot bosses (not shown) of proximal flanges 183a within corresponding openings (not shown) defined within the inner support sleeve 153 and operably coupled with outer drive sleeve 152 via a drive pin 155 secured relative to outer drive sleeve 152 and pivotably received within apertures 183b defined within proximal flanges 183a. As such, sliding of outer drive sleeve 152 about inner support sleeve 153 pivots jaw member 164 relative to blade 162 from the open position towards the closed position to clamp tissue between jaw liner 184 of jaw member 164 and blade 162.

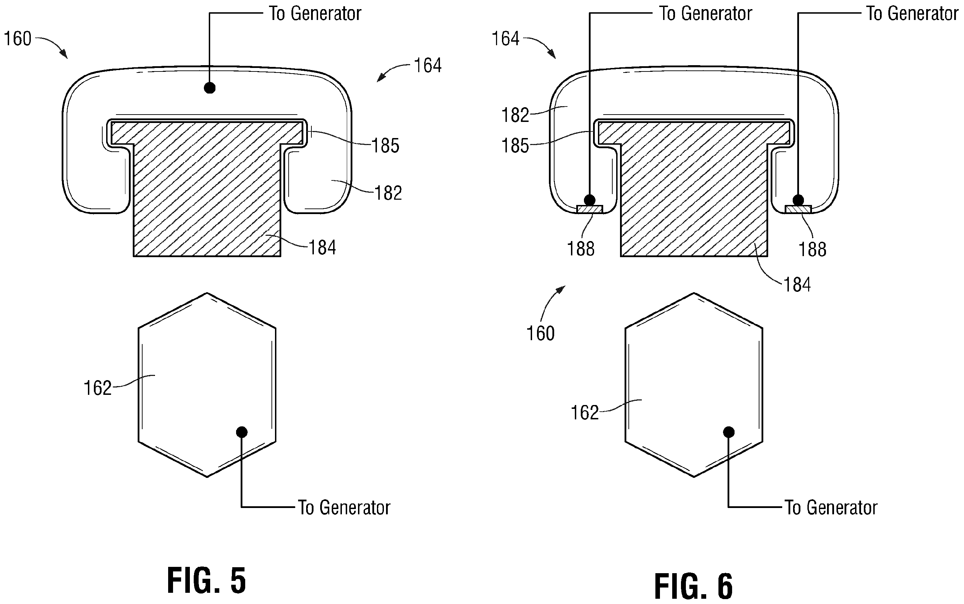

With reference to FIG. 5, structural body 182 may be adapted to connect to a source of electrosurgical energy, e.g., generator 200 (FIG. 1), and, in a bipolar configuration, is charged to a different potential as compared to blade 162 to enable the conduction of bipolar electrosurgical (e.g., RF) energy through tissue clamped therebetween, to treat the tissue. In a monopolar configuration, structural body 182 may be un-energized, may be charged to the same potential as compared to blade 162 (thus both defining the active electrode), or may be energized while blade 162 is not energized (wherein structural body 182 defines the active electrode). In either monopolar configuration, energy is returned to generator 200 (FIG. 1) via return electrode device 500 (FIG. 1), which serves as the passive or return electrode.

Referring to FIG. 6, as an alternative to the entirety of structural body 182 of jaw member 164 being connected to generator 200 (FIG. 1), the structural body may be formed from or embedded at least partially in an insulative material, e.g., an overmolded plastic. In such configurations, electrically conductive surfaces 188, e.g., in the form of plates, may be disposed on or captured by the overmolded plastic to define electrodes on either side of jaw liner 184 on the blade facing side of jaw member 164. The electrically conductive surfaces 188, in such aspects, are connected to generator 200 (FIG. 1) and may be energized for use in bipolar and/or monopolar configurations, e.g., energized to the same potential as one another and/or blade 162 and/or different potentials as one another and/or blade 162. In aspects, electrically conductive surfaces 188 are disposed at additional or alternative locations on jaw member 164, e.g., along either or both sides thereof, along a back surface thereof, etc.

Returning to FIGS. 4-6, jaw liner 184 is shaped complementary to a cavity 185 defined within structural body 182, e.g., defining a T-shaped configuration, to facilitate receipt and retention therein, although other configurations are also contemplated. Jaw liner 184 is fabricated from an electrically insulative, compliant material such as, for example, polytetrafluoroethylene (PTFE). The compliance of jaw liner 184 enables blade 162 to vibrate while in contact with jaw liner 184 without damaging components of ultrasonic surgical instrument 100 (FIG. 1) and without compromising the hold on tissue clamped between jaw member 164 and blade 162. Jaw liner 184 extends from structural body 182 towards blade 162 to inhibit contact between structural body 182 and blade 162 in the closed position of jaw member 164. The insulation of jaw liner 184 maintains electrical isolation between blade 162 and structural body 182 of jaw member 164, thereby inhibiting shorting.

Referring generally to FIGS. 1-6, depending upon a surgical task to be performed and/or other factors, the use of a surgical instrument or system, e.g., surgical instrument 100 (FIG. 1), surgical instrument 20 (FIG. 2), or surgical system 1000 (FIG. 3), may vary. For example, clamping tissue under a clamping force may be required to perform certain tasks, e.g., to seal clamped tissue and/or transect the sealed tissue; while an applied clamping force or even the position of the jaw member may be inconsequential for performing other surgical tasks, e.g., performing an otomy, backscoring, etc. Thus, while it may be important in certain situations to confirm to the user that clamp lever 130 has been actuated at least to the point of sufficient actuation, it may not be advantageous to inhibit, at least in all situations, application of energy when clamp lever 130 has not been actuated at least to the point of sufficient actuation.

In aspects, a suitable output from generator 200, e.g., as detailed above, may be provided to indicate to the user whether or not clamp lever 130 has been actuated at least to the point of sufficient actuation. The user, based upon the surgical task to be performed, may then determine whether actuating the clamp lever 130 at least to the point of sufficient actuation is required or not and, if necessary, take appropriate action, e.g., sufficiently actuate clamp lever 130 to ensure that a clamping force is applied to clamped tissue. This configuration may be advantageous where the user intends to sufficiently actuate the clamp lever 130 but has not actually done so and/or that there is some other unexpected condition, e.g., where tissue has slipped out of the area between jaw member 164 and blade 162 and is no longer clamped.

As an alternative or in addition to providing an output from generator 200, generator 200 may enable/disable the application of energy based upon whether clamp lever 130 has been actuated at least to the point of sufficient actuation and/or other information. For example, where the instrument or system is activated in the first state (such as the LOW power and/or tissue sealing setting), energy application may be inhibited unless and until the clamp lever 130 is actuated at least to the point of sufficient actuation. If clamp lever 130 is actuated at least to the point of sufficient actuation, bipolar energy and/or ultrasonic energy (in a low power mode) are activated (while monopolar energy, if such capability is included, remains off) to enable tissue sealing. In aspects, after tissue sealing, where activation of the instrument or system is moved from the first state to the second state (such as the HIGH power and/or tissue transection setting), and if clamp lever 130 is actuated (or remains actuated) at least to the point of sufficient actuation, bipolar energy and ultrasonic energy (in high power mode), or just ultrasonic energy in the high power mode, are activated (while monopolar energy remains off) to enable transection of the sealed tissue. In other instances, e.g., where the instrument or system is activated in the second state without prior activation in the first state, energy application may proceed regardless of whether clamp lever 130 is actuated at least to the point of sufficient actuation.

Further still, generator 200 may determine the modality(s) of energy to be applied and/or select the parameters of energy delivery (e.g., high versus low power, coagulation versus cutting, etc.) based upon whether clamp lever 130 has been actuated at least to the point of sufficient actuation and/or other information. For example, when it is determined that clamp lever 130 has not been actuated at least to the point of sufficient actuation and that the instrument or system is activated in the first state, bipolar energy is off and monopolar and/or ultrasonic energy are activated. In such aspects, the monopolar energy may be activated in a coagulation mode and/or the ultrasonic energy may be activated in a low power mode. On the other hand, when it is determined that clamp lever 130 has been actuated at least to the point of sufficient actuation and that the instrument or system is activated in the first state, bipolar energy and/or ultrasonic energy are activated while monopolar energy is turned off. In such aspects, the ultrasonic energy may be activated in a low power mode.

As another example, when it is determined that clamp lever 130 has not been actuated at least to the point of sufficient actuation and that the instrument or system is activated in the second state, bipolar energy is off and monopolar and/or ultrasonic energy are activated. In such aspects, the monopolar energy may be activated in a cut mode and/or the ultrasonic energy may be activated in a high power mode. On the other hand, when it is determined that clamp lever 130 has been actuated at least to the point of sufficient actuation and that the instrument or system is activated in the second state, bipolar energy and/or ultrasonic energy are activated while monopolar energy is turned off. In such aspects, ultrasonic energy may be activated in a high power mode.

Other suitable energy control and/or outputs may be provided based upon the determination, e.g., from the one or more sensors 132 (FIG. 1), as to whether clamp lever 130 has been actuated at least to the point of sufficient actuation.

While several aspects of the disclosure have been detailed above and are shown in the drawings, it is not intended that the disclosure be limited thereto, as it is intended that the disclosure be as broad in scope as the art will allow and that the specification be read likewise. Therefore, the above description and accompanying drawings should not be construed as limiting, but merely as exemplifications of particular aspects. Those skilled in the art will envision other modifications within the scope and spirit of the claims appended hereto.

The invention may be described by reference to the following numbered paragraphs:-
1. A surgical system, comprising:
   a housing;
   an ultrasonic transducer supported by the housing;
   an end effector assembly distally-spaced from the housing, the end effector assembly including:
      an ultrasonic blade operably coupled to the ultrasonic transducer for receiving ultrasonic energy produced by the ultrasonic transducer; and
      a jaw member pivotable relative to the blade from an open position towards a clamping position for clamping tissue between the jaw member and the blade and imparting a clamping force to the clamped tissue;
   a clamp lever operably coupled to the housing and the jaw member such that actuation of the clamp lever relative to the housing pivots the jaw member towards the blade; and
   a sensor configured to sense whether the clamp lever is sufficiently actuated so as to impart the clamping force to the clamped tissue.
2. The surgical system according to paragraph 1, further comprising a generator operably coupled to the ultrasonic transducer and configured to control activation of the ultrasonic transducer to generate ultrasonic energy for transmission to the blade.
3. The surgical system according to paragraph 2, wherein the generator is communicatively coupled to the sensor and configured to control activation of the ultrasonic transducer based at least in part on feedback from the sensor indicating whether the clamp lever is sufficiently actuated.
4. The surgical system according to paragraph 3, further comprising at least one activation button configured for activation in each of a first state and a second state, and wherein the generator is configured to control activation of the ultrasonic transducer based at least in part on feedback from the sensor indicating whether the clamp lever is sufficiently actuated and the activation state of the at least one activation button.
5. The surgical system according to paragraph 2, wherein the generator is communicatively coupled to the sensor and configured to provide an output based on feedback from the sensor indicating whether the clamp lever is sufficiently actuated.
6. The surgical system according to paragraph 1, wherein at least one of the jaw member or the blade is adapted to connect to a source of electrosurgical energy for supplying electrosurgical energy to tissue.
7. The surgical system according to paragraph 6, wherein the jaw member and the blade are adapted to connect to the source of electrosurgical energy for conducting bipolar electrosurgical energy therebetween and through tissue.
8. The surgical system according to paragraph 6, wherein at least one of the jaw member or the blade is adapted to connect to the source of electrosurgical energy for supplying monopolar electrosurgical energy through tissue to a remote return device.
9. A surgical system, comprising:
   a housing;
   an ultrasonic transducer supported by the housing;
   an end effector assembly distally-spaced from the housing, the end effector assembly including:
      an ultrasonic blade operably coupled to the ultrasonic transducer for receiving ultrasonic energy produced by the ultrasonic transducer; and
      a jaw member pivotable relative to the blade from an open position towards a clamping position for clamping tissue between the jaw member and the blade and imparting a clamping force to the clamped tissue;
   a clamp lever operably coupled to the housing and the jaw member such that actuation of the clamp lever relative to the housing pivots the jaw member towards the blade;
   a sensor configured to sense whether the clamp lever is sufficiently actuated so as to impart the clamping force to the clamped tissue; and
   a generator operably coupled to the ultrasonic transducer to control activation of the ultrasonic transducer to generate ultrasonic energy for transmission to the blade and communicatively coupled to the sensor, wherein the generator is configured to at least one of:
      provide an output based on feedback from the sensor indicating whether the clamp lever is sufficiently actuated; or
      control activation of the ultrasonic transducer based at least in part on feedback from the sensor indicating whether the clamp lever is sufficiently actuated.
10. The surgical system according to paragraph 9, further comprising at least one activation button configured for activation in each of a first state and a second state, and wherein the surgical generator is configured to control activation of the ultrasonic transducer based at least in part on feedback from the sensor indicating whether the clamp lever is sufficiently actuated and the activation state of the at least one activation button.
11. The surgical system according to paragraph 9, wherein the generator is configured to supply electrosurgical energy to at least one of the jaw member or the blade for supplying the electrosurgical energy to tissue.
12. The surgical system according to paragraph 11, wherein the jaw member and the blade are configured to conduct bipolar electrosurgical energy therebetween and through tissue.
13. The surgical system according to paragraph 11, wherein at least one of the jaw member or the blade is configured to supply monopolar electrosurgical energy through tissue to a remote return device for return to the generator.
14. The surgical system according to paragraph 11, wherein the generator is configured to control activation of the ultrasonic transducer and the supply of electrosurgical energy based at least in part on feedback from the sensor indicating whether the clamp lever is sufficiently actuated.

## Claims

1. A surgical system, comprising:
a housing;
an ultrasonic transducer supported by the housing;
an end effector assembly distally-spaced from the housing, the end effector assembly including:
an ultrasonic blade operably coupled to the ultrasonic transducer for receiving ultrasonic energy produced by the ultrasonic transducer; and
a jaw member pivotable relative to the blade from an open position towards a clamping position for clamping tissue between the jaw member and the blade and imparting a clamping force to the clamped tissue;
a clamp lever operably coupled to the housing and the jaw member such that actuation of the clamp lever relative to the housing pivots the jaw member towards the blade; and
a sensor configured to sense whether the clamp lever is sufficiently actuated so as to impart the clamping force to the clamped tissue.

2. The surgical system according to claim 1, further comprising a generator operably coupled to the ultrasonic transducer and configured to control activation of the ultrasonic transducer to generate ultrasonic energy for transmission to the blade.

3. The surgical system according to claim 2, wherein the generator is communicatively coupled to the sensor and configured to control activation of the ultrasonic transducer based at least in part on feedback from the sensor indicating whether the clamp lever is sufficiently actuated.

4. The surgical system according to claim 3, further comprising at least one activation button configured for activation in each of a first state and a second state, and wherein the generator is configured to control activation of the ultrasonic transducer based at least in part on feedback from the sensor indicating whether the clamp lever is sufficiently actuated and the activation state of the at least one activation button.

5. The surgical system according to claim 2, wherein the generator is communicatively coupled to the sensor and configured to provide an output based on feedback from the sensor indicating whether the clamp lever is sufficiently actuated.

6. The surgical system according to any preceding claim, wherein at least one of the jaw member or the blade is adapted to connect to a source of electrosurgical energy for supplying electrosurgical energy to tissue.

7. The surgical system according to claim 6, wherein the jaw member and the blade are adapted to connect to the source of electrosurgical energy for conducting bipolar electrosurgical energy therebetween and through tissue.

8. The surgical system according to claim 6, wherein at least one of the jaw member or the blade is adapted to connect to the source of electrosurgical energy for supplying monopolar electrosurgical energy through tissue to a remote return device.

9. A surgical system, comprising:
a housing;
an ultrasonic transducer supported by the housing;
an end effector assembly distally-spaced from the housing, the end effector assembly including:
an ultrasonic blade operably coupled to the ultrasonic transducer for receiving ultrasonic energy produced by the ultrasonic transducer; and
a jaw member pivotable relative to the blade from an open position towards a clamping position for clamping tissue between the jaw member and the blade and imparting a clamping force to the clamped tissue;
a clamp lever operably coupled to the housing and the jaw member such that actuation of the clamp lever relative to the housing pivots the jaw member towards the blade;
a sensor configured to sense whether the clamp lever is sufficiently actuated so as to impart the clamping force to the clamped tissue; and
a generator operably coupled to the ultrasonic transducer to control activation of the ultrasonic transducer to generate ultrasonic energy for transmission to the blade and communicatively coupled to the sensor, wherein the generator is configured to at least one of:
provide an output based on feedback from the sensor indicating whether the clamp lever is sufficiently actuated; or
control activation of the ultrasonic transducer based at least in part on feedback from the sensor indicating whether the clamp lever is sufficiently actuated.

10. The surgical system according to claim 9, further comprising at least one activation button configured for activation in each of a first state and a second state, and wherein the surgical generator is configured to control activation of the ultrasonic transducer based at least in part on feedback from the sensor indicating whether the clamp lever is sufficiently actuated and the activation state of the at least one activation button.

11. The surgical system according to claim 9, wherein the generator is configured to supply electrosurgical energy to at least one of the jaw member or the blade for supplying the electrosurgical energy to tissue.

12. The surgical system according to claim 11, wherein the jaw member and the blade are configured to conduct bipolar electrosurgical energy therebetween and through tissue.

13. The surgical system according to claim 11, wherein at least one of the jaw member or the blade is configured to supply monopolar electrosurgical energy through tissue to a remote return device for return to the generator.

14. The surgical system according to claim 11,12 or 13 wherein the generator is configured to control activation of the ultrasonic transducer and the supply of electrosurgical energy based at least in part on feedback from the sensor indicating whether the clamp lever is sufficiently actuated.
